# EUROPÄISCHE PATENTANMELDUNG

(11) **EP 2 949 646 A1**
(43) Veröffentlichungstag der Anmeldung: **02.12.2015**
(21) Anmeldenummer: 15168645.8
(22) Anmeldetag: 21.05.2015
(51) Int. Cl.: C07C 391/02

(54) **VERFAHREN ZUR HERSTELLUNG VON 2,2'-SELENOBIARYLETHERN ODER 4,4'-SELENOBIARYLETHERN UNTER VERWENDUNG VON SELENDIOXID**

(30) Priorität: 26.05.2014 DE 102014209974
(71) Anmelder: Evonik Degussa GmbH, 45128 Essen (DE)
(72) Erfinder: Dyballa, Katrin Marie, 45657 Recklinghausen (DE); Franke, Robert, 45772 Marl (DE); Fridag, Dirk, 45721 Haltern am See (DE); Waldvogel, Siegfried R., 55435 Gau-Algesheim (DE); Quell, Thomas, 55118 Mainz (DE); Mirion, Michael, 55122 Mainz (DE)

(57) **Zusammenfassung**

Verfahren zur Herstellung von 2,2'-Selenobiarylethern oder 4,4'-Selenobiarylethern unter Verwendung von Selendioxid.

## Beschreibung

Die Erfindung betrifft ein Verfahren zur Herstellung von 2,2'-Selenobiarylethern oder 4,4'-Selenobiarylethern unter Verwendung von Selendioxid, sowie einen neuen 2,2'-Selenobiarylether oder 4,4'-Selenobiarylether.

2,2'-Selenobiarylether oder 4,4'-Selenobiarylether stellen eine hochinteressante und vielversprechende Verbindungsklasse dar. Diese Verbindungen werden derzeit in bestimmte Komplexe, vor allem manganhaltig, eingebaut, besitzen aber ein großes Potential für weitere Anwendungen.

Der Begriff Phenole wird in dieser Anmeldung als Gattungsbegriff verwendet und umfasst somit auch substituierte Phenole.

T. K. Paine beschreibt eine Synthese von 2,2'-Selenobis(4,6-di-tert-butylphenol) unter Verwendung von Selendioxid. Die Herstellung von 2,2'-Selenobis(4,6-di-tert-butylphenol) erfolgt hier in einem sauren Medium unter Zusatz von konzentrierter Salzsäure. Das Produkt wird mit einer Ausbeute von 25 % erhalten (T. K. Paine et al., "Manganese complexes of mixed O, X, O-donor ligands (X = S or Se): synthesis, characterization and catalytic reactivity", Dalton Trans., 2003, 15, 3136-3144).
Hierbei ist besonders nachteilig, dass die Ausbeuten sehr gering und somit verbesserungswürdig sind.

H. M. Lin beschreibt eine Syntheseroute für Selenobiarylether, welche über mehrere Stufen erfolgt. Zuerst muss Brom an das entsprechende Phenol addiert werden, um das Produkt dann mit Magnesium zu einem Grignard-Reagenz umzusetzen. Das Grignard-Reagenz kann dann mit dem zugesetzten Selen reagieren, bevor die eigentliche Kupplung zum Biarylether erfolgt: 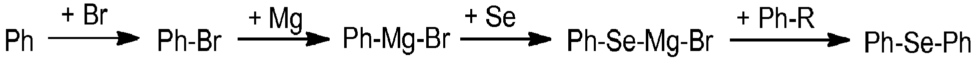 (H. M. Lin et al., "A novel and efficient synthesis of selenides", ARKIVOC, 2012, viii, 146-156)

Das Produkt konnte in einer guten Ausbeute erzielt werden, jedoch ist diese Syntheseroute sehr aufwendig, was es für einen großtechnischen Einsatz unattraktiv macht. Hierbei sind eine Vielzahl von Syntheseschritten notwendig, die zum Teil nicht unkritisch in ihrer Durchführung sind, insbesondere wenn man an eine Hochskalierung denkt und Maßstäbe verwendet, die in der Industrie üblich sind. Weiterhin fallen bei dieser Syntheseroute große Mengen an Abfallprodukten und Lösungsmittel an, die aufwändig entsorgt werden müssen, unter anderem auf Grund des Einsatzes von Brom.

Der Erfindung lag die Aufgabe zugrunde, ein Verfahren bereitzustellen, welches die in Zusammenhang mit dem Stand der Technik beschriebenen Nachteile nicht aufweist. Insbesondere soll ein Verfahren bereitgestellt werden, mit welchem 2,2'-Selenobiarylether oder 4,4'-Selenobiarylether selektiv hergestellt werden können, also bei der Herstellung möglichst wenig Nebenprodukte anfallen. Das Verfahren sollte auch großtechnisch einsetzbar sein, und deshalb möglichst wenig Einzelschritte und Zwischenstufen aufweisen.

Gelöst wird die Aufgabe durch ein Verfahren nach Anspruch 1.

Verfahren zur Herstellung von 2,2'-Selenobiarylethern oder 4,4'-Selenobiarylethern umfassend die Verfahrensschritte:
a) Zugabe eines ersten Phenols zum Reaktionsgemisch,
b) Zugabe eines zweiten Phenols zum Reaktionsgemisch,
c) Zugabe von Selendioxid zum Reaktionsgemisch,
d) Zugabe einer Base mit einem pKb-Wert im Bereich von 8 bis 11 zum Reaktionsgemisch,
e) Einstellen der Reaktionstemperatur des Reaktionsgemisches, so dass das erste Phenol und das zweite Phenol zu einem 2,2'-Selenobiarylether oder 4,4'-Selenobiarylether umgesetzt werden.

Die Schritte a) bis d) können hierbei in beliebiger Reihenfolge durchgeführt werden.

Das Verfahren ist nicht auf die oben dargestellten Komponenten beschränkt. Weitere Bestandteile wie beispielsweise Lösungsmittel können ebenfalls in dem Reaktionsgemisch enthalten sein.

Verfügt die Base über mehr als einen pKb-Wert, so ist der pKb₁-Wert heranzuziehen. Dieser muss im erfindungsgemäßen Fall im Bereich von 8 bis11 liegen. Die Definition des pKs-Wertes bzw. pKb-Wertes ist dem Fachmann hinlänglich bekannt und kann der entsprechenden Fachliteratur entnommen werden.

Ein Problem beim Einsatz von Selendioxid ist, dass 2,2'-Biphenole und das entsprechende Pummerer-Keton in großen Mengen als Nebenprodukt anfallen können. Bei ungünstiger Reaktionsführung kann es sogar vorkommen, dass 2,2'-Biphenole das Hauptprodukt der Reaktion darstellt. Gemäß der Aufgabenstellung der Erfindung gilt es jedoch die Reaktion gerade so zu führen, dass jene Nebenprodukte möglichst reduziert werden.

Durch Zugabe von Selendioxid als Oxidationsmittel können abhängig von den Reaktionsbedingungen 2,2'-Biphenole oder 2,2'-Selenobiarylether als Hauptprodukte der Reaktion anfallen (vgl. Schema 1).

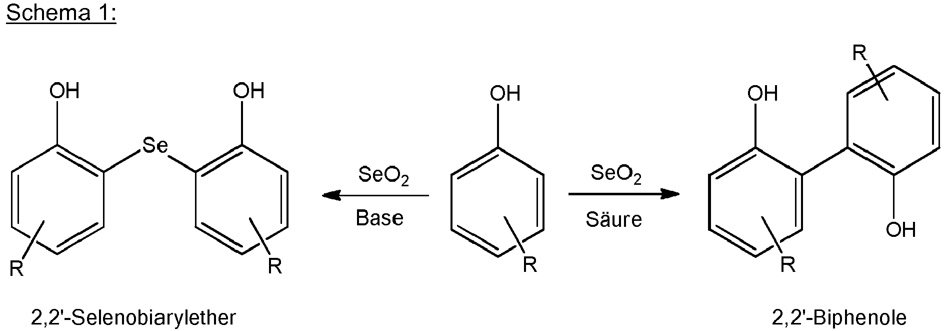

Es wurde gefunden, dass sich die Reaktion durch Zugabe einer Base mit einem pKb-Wert im Bereich von 8 bis 11 gezielt in die Richtung des 2,2'-Selenobiarylether verschieben lässt.

Weitere Vorteile gegenüber der im Stand der Technik beschriebenen Verfahren sind, dass dabei nicht unter Feuchtigkeits- oder Sauerstoffausschluss gearbeitet werden muss. Dies stellt einen deutlichen Vorteil gegenüber anderen Syntheserouten dar. Diese Verfahren hebt sich in vorteilhafter Weise von den bestehenden mehrstufigen Syntheserouten abhebt.

Über den pKb-Werte kann die Reaktion in Richtung 2,2'-Selenobiarylether gelenkt werden. Dadurch, dass überwiegend das gewünschte Hauptprodukt gebildet wird und die Bildung von höhermolekularen Überoxidationsprodukten verringert wird, wird die Aufarbeitung deutlich vereinfacht.

Nicht umgesetzte Edukte sowie eingesetzte Lösungsmittel können destillativ zurückgewonnen und für weitere Reaktionen verwendet werden. Somit erfüllt das erfindungsgemäße Verfahren die Erfordernisse eines ökonomischen, großtechnischen Verfahrens.

Ferner wird im erfindungsgemäßen Verfahren Selendioxid verwendet. Selendioxid ist ein Abfallprodukt aus der Metallaufreinigung und Erzraffination. Somit wird in dem hier beanspruchten Verfahren ein Abfallprodukt aus anderen Prozessen erneut wertschöpfend eingesetzt. Dies ist insbesondere vor dem Hintergrund der Nachhaltigkeit von Prozessen ein wichtiges Thema.

In einer Variante des Verfahrens handelt es sich bei dem ersten Phenol in Verfahrensschritt a) um eine Verbindung gemäß der allgemeinen Formel **I:** 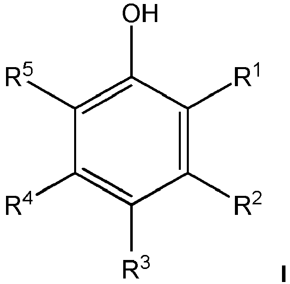 wobei R¹, R², R³, R⁴, R⁵ jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen (wie Cl, F, Br, I), -OC=O-(C₁-C₁₂)-Alkyl,
zwei benachbarte Reste können des Weiteren zu einem kondensierten System miteinander verbunden sein,
wobei die genannten Alkyl- und Arylgruppen substituiert sein können, und mindestens R¹ oder R⁵ gleich -H ist.

Mit diesem Verfahren wird ein 2,2'-Selenobiarylethern hergestellt.
(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind aus:
(C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl.

(C₆-C₂₀)-Aryl und O-(C₆-C₂₀)-Aryl können jeweils unsubstituiert oder durch einen oder mehrere gleiche oder verschiedene Reste substituiert sein, die ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂-

Im Rahmen der Erfindung umfasst der Ausdruck (C₁-C₁₂)-Alkyl geradkettige und verzweigte Alkylgruppen. Vorzugsweise handelt es sich dabei um unsubstituierte geradkettige oder verzweigte (C₁-C₈)-Alkylgruppen und ganz bevorzugt (C₁-C₆)-Alkylgruppen. Beispiele für (C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Dimethylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte (C₁-C₁₂)-Alkylgruppen und substituierte (C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt sind aus:
-(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

In einer Variante des Verfahrens sind R¹, R², R³, R⁴, R⁵ jeweils unabhängig voneinander ausgewählt aus:
-H, -(C₁-C₁₂)-Alkyl, -O-(C₁-C₁₂)-Alkyl, -(C₆-C₂₀)-Aryl, -O-(C₆-C₂₀)-Aryl,
wobei die genannten Alkyl- und Arylgruppen substituiert sein können, und mindestens R¹ oder R⁵ gleich -H ist.

In einer Variante des Verfahrens sind R¹, R², R³, R⁴, R⁵ jeweils unabhängig voneinander ausgewählt aus:
-H, -(C₁-C₁₂)-Alkyl,
wobei die genannten Alkyl- und Arylgruppen substituiert sein können,
und mindestens R¹ oder R⁵ gleich -H ist.

In einer Variante des Verfahrens sind R¹, R³, R⁵ jeweils unabhängig voneinander ausgewählt aus:
-H, -(C₁-C₁₂)-Alkyl,
wobei die genannten Alkylgruppen substituiert sein können,
und mindestens R¹ oder R⁵ gleich -H ist.

In einer Variante des Verfahrens stehen R² und R⁴ für -H.

In einer Variante des Verfahrens handelt es sich bei dem zweiten Phenol in Verfahrensschritt b) um eine Verbindung gemäß der allgemeinen Formel **II**: 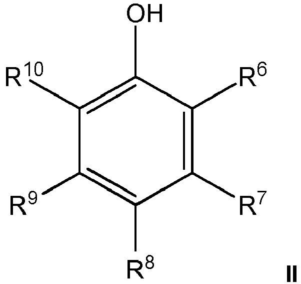 wobei R⁶, R⁷, R⁸, R⁹, R¹⁰ jeweils unabhängig voneinander ausgewählt sind aus:
- H, -(C₁-C₁₂)-Alkyl, -O-(C₁-C₁₂)-Alkyl, -(C₆-C₂₀)-Aryl, -O-(C₆-C₂₀)-Aryl, -Halogen (wie Cl, F, Br, I), -OC=O-(C₁-C₁₂)-Alkyl,
zwei benachbarte Reste können des Weiteren zu einem kondensierten System miteinander verbunden sein,
wobei die genannten Alkyl- und Arylgruppen substituiert sein können, und mindestens R⁶ oder R¹⁰ gleich -H ist.

In einer Variante des Verfahrens sind R⁶, R⁷, R⁸, R⁹, R¹⁰ jeweils unabhängig voneinander ausgewählt aus:
-H, -(C₁-C₁₂)-Alkyl, -O-(C₁-C₁₂)-Alkyl, -(C₆-C₂₀)-Aryl, -O-(C₆-C₂₀)-Aryl,
wobei die genannten Alkyl- und Arylgruppen substituiert sein können,
und mindestens R⁶ oder R¹⁰ gleich -H ist.

In einer Variante des Verfahrens sind R⁶, R⁷, R⁸, R⁹, R¹⁰ jeweils unabhängig voneinander ausgewählt aus:
-H, -(C₁-C₁₂)-Alkyl,
wobei die genannten Alkyl- und Arylgruppen substituiert sein können,
und mindestens R⁶ oder R¹⁰ gleich -H ist.

In einer Variante des Verfahrens sind R⁶, R⁸, R¹⁰ jeweils unabhängig voneinander ausgewählt aus:
-H, -(C₁-C₁₂)-Alkyl,
   wobei die genannten Alkylgruppen substituiert sein können,
   und mindestens R⁶ oder R¹⁰ gleich -H ist.

In einer Variante des Verfahrens stehen R⁷ und R⁹ für -H.

In einer Variante des Verfahrens entspricht das erste Phenol dem zweiten Phenol.
Bei dieser Variante handelt es sich also um eine Homokupplung zweier gleicher Phenole, welche über das Selen verknüpft werden.

In einer Variante des Verfahrens wird das Selendioxid in Verfahrensschritt c) in einem molaren Verhältnis bezogen auf die Summe des ersten und des zweiten Phenols zugegeben, welches in einem Bereich von 0,25 bis 1,5 liegt.
Hierbei ist der Bereich von 0,25 bis 0,9 bevorzugt, und der Bereich von 0,4 bis 0,7 besonders bevorzugt.

In einer Variante des Verfahrens ist die Base im Verfahrensschritt d) ausgewählt aus:
Pyridin, Chinolin.

Hierbei ist Pyridin bevorzugt.

In einer Variante des Verfahrens wird die Base im Verfahrensschritt d) als Lösungsmittel eingesetzt.

In einer Variante des Verfahrens wird das Reaktionsgemisch im Verfahrensschritt e) auf eine Temperatur in dem Bereich von 0 °C bis 100 °C eingestellt.
Hierbei ist der Bereich von 20 °C bis 90 °C bevorzugt, und der Bereich von 30 °C bis 80 °C besonders bevorzugt.

In einer Variante des Verfahrens wird die im Verfahrensschritt e) eingestellte Temperatur über einen Zeitraum im Bereich von 1 Stunde bis 48 Stunden gehalten.
Hierbei ist der Bereich von 1 Stunde bis 24 Stunden bevorzugt, und der Bereich von 2 Stunden bis 10 Stunden besonders bevorzugt.

Neben dem Verfahren wird auch eine neuer 2,2'-Selenobiarylether beansprucht.

Verbindung gemäß der Formel:

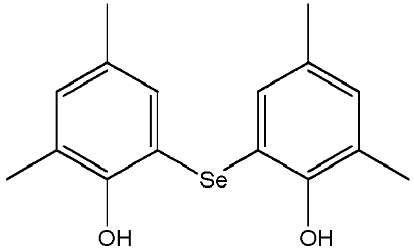

In einer Variante des Verfahrens handelt es sich bei dem ersten Phenol in Verfahrensschritt a) um eine Verbindung gemäß der allgemeinen Formel **III**: 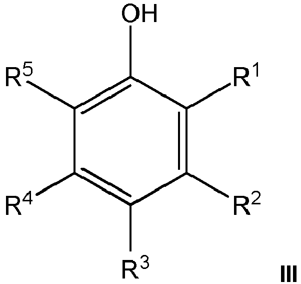 wobei R¹, R², R³, R⁴, R⁵ jeweils unabhängig voneinander ausgewählt sind aus:
- H, -(C₁-C₁₂)-Alkyl, -O-(C₁-C₁₂)-Alkyl, -(C₆-C₂₀)-Aryl, -O-(C₆-C₂₀)-Aryl, -Halogen (wie Cl, F, Br, I), -OC=O-(C₁-C₁₂)-Alkyl,
zwei benachbarte Reste können des Weiteren zu einem kondensierten System miteinander verbunden sein,
wobei die genannten Alkyl- und Arylgruppen substituiert sein können,
und R³ gleich -H ist.

Mit diesem Verfahren wird ein 4,4'-Selenobiarylethern hergestellt.

In einer Variante des Verfahrens sind R¹, R², R³, R⁴, R⁵ jeweils unabhängig voneinander ausgewählt aus:
-H, -(C₁-C₁₂)-Alkyl, -O-(C₁-C₁₂)-Alkyl, -(C₆-C₂₀)-Aryl, -O-(C₆-C₂₀)-Aryl,
wobei die genannten Alkyl- und Arylgruppen substituiert sein können,
und R³ gleich -H ist.

In einer Variante des Verfahrens sind R¹, R², R³, R⁴, R⁵ jeweils unabhängig voneinander ausgewählt aus:
-H, -(C₁-C₁₂)-Alkyl,
wobei die genannten Alkyl- und Arylgruppen substituiert sein können, und R³ gleich -H ist.

In einer Variante des Verfahrens sind R¹, R³, R⁵ jeweils unabhängig voneinander ausgewählt aus:
-H, -(C₁-C₁₂)-Alkyl,
wobei die genannten Alkylgruppen substituiert sein können,
und R³ gleich -H ist.

In einer Variante des Verfahrens stehen R² und R⁴ für -H.

In einer Variante des Verfahrens handelt es sich bei dem zweiten Phenol in Verfahrensschritt b) um eine Verbindung gemäß der allgemeinen Formel **IV**: 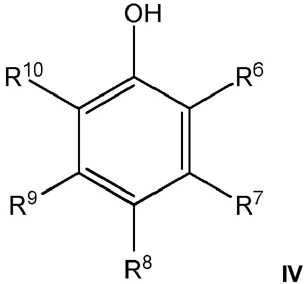 wobei R⁶, R⁷, R⁸, R⁹, R¹⁰ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Halogen (wie Cl, F, Br, I), -OC=O-(C₁-C₁₂)-Alkyl,
zwei benachbarte Reste können des Weiteren zu einem kondensierten System miteinander verbunden sein,
wobei die genannten Alkyl- und Arylgruppen substituiert sein können,
und R⁸ gleich -H ist.

In einer Variante des Verfahrens sind R⁶, R⁷, R⁸, R⁹, R¹⁰ jeweils unabhängig voneinander ausgewählt aus:
-H, -(C₁-C₁₂)-Alkyl, -O-(C₁-C₁₂)-Alkyl, -(C₆-C₂₀)-Aryl, -O-(C₆-C₂₀)-Aryl,
wobei die genannten Alkyl- und Arylgruppen substituiert sein können,
und R⁸ gleich -H ist.

In einer Variante des Verfahrens sind R⁶, R⁷, R⁸, R⁹, R¹⁰ jeweils unabhängig voneinander ausgewählt aus:
-H, -(C₁-C₁₂)-Alkyl,
wobei die genannten Alkyl- und Arylgruppen substituiert sein können,
und R⁸ gleich -H ist.

In einer Variante des Verfahrens sind R⁶, R⁸, R¹⁰ jeweils unabhängig voneinander ausgewählt aus:
-H, -(C₁-C₁₂)-Alkyl,
wobei die genannten Alkylgruppen substituiert sein können,
und R⁸ gleich -H ist.

In einer Variante des Verfahrens stehen R⁷ und R⁹ für -H.

In einer Variante des Verfahrens entspricht das erste Phenol dem zweiten Phenol.
Bei dieser Variante handelt es sich also um eine Homokupplung zweier gleicher Phenole, welche über das Selen verknüpft werden.

In einer Variante des Verfahrens wird das Selendioxid in Verfahrensschritt c) in einem molaren Verhältnis bezogen auf die Summe des ersten und des zweiten Phenols zugegeben, welches in einem Bereich von 0,25 bis 1,5 liegt.
Hierbei ist der Bereich von 0,25 bis 0,9 bevorzugt, und der Bereich von 0,4 bis 0,7 besonders bevorzugt.

In einer Variante des Verfahrens ist die Base im Verfahrensschritt d) ausgewählt aus:
Pyridin, Chinolin.
Hierbei ist Pyridin bevorzugt.

In einer Variante des Verfahrens wird die Base im Verfahrensschritt d) als Lösungsmittel eingesetzt.

In einer Variante des Verfahrens wird das Reaktionsgemisch im Verfahrensschritt e) auf eine Temperatur in dem Bereich von 0 °C bis 100 °C eingestellt.
Hierbei ist der Bereich von 20 °C bis 90 °C bevorzugt, und der Bereich von 30 °C bis 80 °C besonders bevorzugt.

In einer Variante des Verfahrens wird die im Verfahrensschritt e) eingestellte Temperatur über einen Zeitraum im Bereich von 1 Stunde bis 48 Stunden gehalten.

Hierbei ist der Bereich von 1 Stunde bis 24 Stunden bevorzugt, und der Bereich von 2 Stunden bis 10 Stunden besonders bevorzugt.

Neben dem Verfahren wird auch eine neuer 4,4'-Selenobiarylether beansprucht.

Verbindung gemäß der Formel:

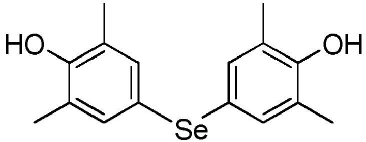

Im Folgenden wird die Erfindung anhand von Ausführungsbeispielen näher erläutert.

### Analytik

### NMR-Spektroskopie

Die NMR-spektroskopischen Untersuchungen wurden an Multikernresonanzspektrometern des Typs AC 300 oder AV II 400 der Firma *Bruker,* Analytische Messtechnik, Karlsruhe, durchgeführt. Als Lösungsmittel wurde CDCl₃ verwendet. Die ¹H- und ¹³C-Spektren wurden gemäß dem Restgehalt an nicht deuteriertem Lösungsmittel nach der *NMR Solvent Data Chart* der Fa. *Cambridge Isotopes Laboratories,* USA, kalibriert. Die Zuordnung der ¹H- und ¹³C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht mit der IUPAC-Nomenklatur übereinstimmen muss.

### Allgemeine Arbeitsvorschrift

8.2 mmol des jeweiligen Phenols werden im entsprechenden Lösungsmittel gelöst (8.2 m). Das Reaktionsgemisch wird erhitzt und 4.9 mmol Selendioxid werden unter rühren zugegeben. Das Lösungsmittel wird im Vakuum destilliert (Temperatur <70° C). Eine Fritte wird mit 2,5 cm Kieselgel (unten) und 2,5 cm Zeolith (oben) vorbereitet. Der Destillationsrückstand wird im Laufmittel aufgenommen und auf die Filtrationssäule geben. Mit Cyclohexan:Essigester (95:5) wird das Produkt von der Fritte waschen und in Fraktionen gesammelt. Die Fraktionen die Produkt enthalten werden zusammengefasst und destillativ vom Laufmittel befreit.

Die erhaltenen Fraktionen werden aus Cyclohexan:Essigester 95:5 umkristallisiert. Dazu wird der feste Rückstand bei 50 °C gelöst und unlösliche Rückstände über eine Glasfritte abfiltriert. Aus der gesättigten Lösung kristallisiert das Reaktionsprodukt bei Raumtemperatur über Nacht. Die erhaltenen Kristalle werden nochmal mit kaltem Cyclohexan gewaschen.

Die Strukturformel zeigt jeweils das bei der Reaktion angefallene Hauptprodukt.

### 3,3',5,5'-Tetramethylbiphenyl-2,2'-diol

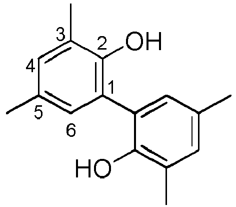

Die Durchführung der Reaktion erfolgt gemäß der allgemeinen Arbeitsvorschrift in einem verschraubbaren Reagenzglas. Hierzu werden 1.00 g (8.2 mmol, 1.0 Äquiv.) 2,4-Dimethylphenol und 0.54 g (4.9 mmol, 0.6 Äquiv.) Selendioxid in 1 mL Säure gelöst und erhitzt. Das Produkt wird als beiger kristalliner Feststoff erhalten.
¹H-NMR (300 MHz, CDCl₃):
δ (ppm) = 7.00 (s,2H, 6-H), 6.87 (s, 2H, 4-H), 5.07 (s,2H, OH), 2.27 (s, 12H, 3-CH₃, 5-CH₃). ¹³C-NMR (75 MHz, CDCl₃):
δ (ppm) = 149.2 (C-2),132.1 (C-4), 130.0 (C-5), 128.5 (C-6), 125.1 (C-3), 122.1 (C-1), 20.4 (5-CH₃), 16.2 (3-CH₃).

### Bis(3,5-dimethyl-2-hydroxyphenyl)selen

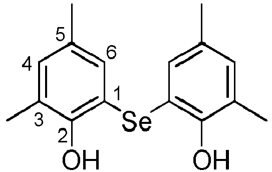

Die Durchführung der Reaktion erfolgt gemäß der allgemeinen Arbeitsvorschrift in einem verschraubbaren Reagenzglas. Hierzu werden 1.00 g (8.2 mmol, 1.0 Äquiv.) 2,4-Dimethylphenol und 0.54 g (4.9 mmol, 0.6 Äquiv.) Selendioxid in 1 mL Pyridin gelöst und erhitzt. Das Produkt wird als farbloser kristalliner Feststoff erhalten.

In einem 250 mL Rundkolben wurden 49.9 g Selendioxid (413 mmol) in 100 mL Pyridin mit Hilfe eines Ölbades auf 55 °C erwärmt. Anschließen wurden 25 mL 2,4-Dimethylphenol (206 mmol) hinzugegeben und die Temperatur für siebeneinhalb Stunden gehalten. Nach Beendigung der Reaktion wurde das Gemisch mit 400 mL Ethylacetat verdünnt und filtriert. Die organische Phase wurde mit Wasser gewaschen und über Magnesiumsulfat getrocknet. Das Pyridin wurden destillativ entfernt und der Rückstand erneut in Ethylacetat gelöst und mit 10%iger Salzsäure und Wasser gewaschen um Resten von Pyridin zu entfernen. Die organische Phase wurde mit Magnesiumsulfat getrocknet und unter vermindertem Druck vom Lösungsmittel befreit. Das so erhaltene Rohprodukt wurde in 400 mL Cyclohexan unter Rückfluss erhitzt. Nach Abkühlung auf Raumtemperatur kristallisierte das Produkt. Nach einem Tag wurde das Produkt abfiltriert, das Filtrat auf die Hälfte eingeengt um bei 4 °C erneut zur Kristallisation gebracht.
Ausbeute: 18.559 g (57.8 mmol), 56 %
¹H-NMR (400 MHz, CDCl₃):
δ (ppm) = 7.12 (s,2H, 6-H), 6.91 (s, 2H, 4-H), 5.97 (s,2H, OH), 2.23 (s, 6H, 3-CH₃) 2.23 (s, 6H, 5-CH₃).
¹³ C-NMR (100 MHz, CDCl₃):
δ (ppm) = 151.7 (C-2),133.2 (C-3), 133.1 (C-5), 130.4 (C-4), 124.2 (C-6), 114.9 (C-1), 20.3 (5-CH₃), 16.5 (3-CH₃).
⁷⁷Se-NMR (76 MHz, CDCl₃):
δ (ppm) = 163.36 ppm.

### Bis(3-tert-butyl-5-methyl-2-hydroxyphenyl)selen

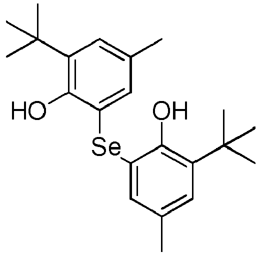

Die Durchführung der Reaktion erfolgt gemäß der allgemeinen Arbeitsvorschrift in einem verschraubbaren Reagenzglas. Dazu wurden 1.32 g (8.0 mmol, 1.0 Äquiv.) 2-*tert*-Butyl-4-methylphenol und 0.54 g (4.9 mmol, 0.6 Äquiv.) Selendioxid in 1 mL Pyridin gelöst und erhitzt.

¹H-NMR (300 MHz, CDCl₃):
δ (ppm) = 7.15 (s, 2H, 6-H), 7.05 (s, 2H, 4-H), 5.07 (s,2H, OH), 2.21 (s, 6H, 5-CH₃), 2.21 (s, 18H, 3-C(CH₃)₃.
¹³C-NMR (75 MHz, CDCl₃):
δ (ppm) = 152.1, 136.4, 133.4, 120.1, 129.5, 117.2, 35.1, 29.6, 20.8.

### 3,3'-Di-tert-butyl,5,5'-dimethylbiphenyl-2,2'-diol

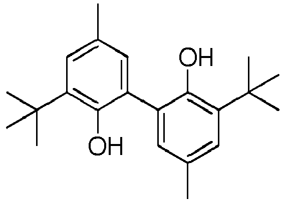

Die Durchführung der Reaktion erfolgt gemäß der allgemeinen Arbeitsvorschrift in einem verschraubbaren Reagenzglas. Dazu wurden 5.00 g (30.5 mmol, 1.0 Äquiv.) 2-*tert*-Butyl-4-methylphenol und 2.03 g (18.3 mmol, 0.6 Äquiv.) Selendioxid in 5 mL Essigsäure gelöst und erhitzt.

¹H-NMR (400 MHz, CDCl₃):
δ (ppm) = 7.17 (d, J=2.2 Hz, 2H), 6.91 (d, J=2.2 Hz, 2H), 5.19 (s, 2H), 2.33 (s, 6H), 1.45 (s, 18H).
¹³C-NMR (75 MHz, CDCl₃):
δ (ppm) = 149.9, 137.0, 129.7, 128.9, 128.6, 122.7, 35.0, 29.8, 27.0.

### Bis(3,5-Di-tert-butyl-2-hydroxyphenyl)selen

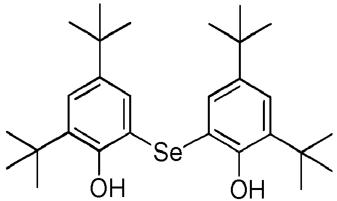

Die Durchführung der Reaktion erfolgt gemäß der allgemeinen Arbeitsvorschrift in einem verschraubbaren Reagenzglas. Dazu wurden 1.67 g (8.2 mmol, 1.0 Äquiv.) *2,4-Di-tert-*butylphenol und 0.55 g (4.9 mmol, 0.6 Äquiv.) Selendioxid in 1 mL Pyridin gelöst und erhitzt.

¹H-NMR (400 MHz, CDCl₃):
δ (ppm) = 7.31 (d, J=2.4 Hz, 2H), 7.29 (d, J=2.4), 6.29 (s, 2H), 1.42 (s, 18H), 1.24 (s, 18H). ¹³C-NMR (75 MHz, CDCl₃):
δ (ppm) = 151.7, 143.5, 135.8, 129.8, 125.6, 117.2, 35.4, 34.4, 31.6, 29.7.

### Bis(3-Chlor-6-hydroxy-5-isopropyl-2-methylphenyl)selen

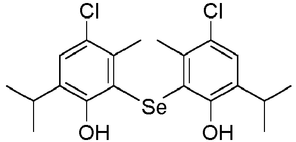

In einem 10 mL Rundkolben wurden 1.0 g Chlorthymol (54 mmol) in 7.5 mL Pyridin gelöst, mit 0.601 g Selendioxid (54 mmol) versetzt und im Ölbad auf 55 °C erhitzt. Nach sieben Tagen wurde die Reaktionslösung mit 50 mL Ethylacetat verdünnt und filtriert. Die organische Phase wurde zunächst zwei Mal mit je 40 mL 10%iger Salzsäure, zwei Mal mit je 40 mL Wasser gewaschen und über Magnesiumsulfat getrocknet. Das, nach Destillation des Lösungsmittels erhaltene, Rohprodukt wurde mittels Säulenchromatographie gereinigt: Die Länge der Säule betrug 10 cm mit einem Durchmesser von 4 cm. Als Eluent wurde Cyclohexan/Essigsäureethylester im Verhältnis 95/5 verwendet. Das so erhaltene Produkt wurde in 20 mL Cyclohexan unter Rückfluss erhitzt. Eine gute Kristallisation konnte nur durch sehr langsames Abkühlen im Ölbad erreicht werden. Das Produkt wurde abfiltriert und mit kaltem Cyclohexan gewaschen. Das Filtrat wurde eingeengt und bei 4 °C innerhalb von zwei Tagen erneut zur Kristallisation gebracht. Nach abfiltrieren des Feststoffes wurde das Filtrat erneut eingeengt und bei 4 °C für sieben Tage zur Kristallisation gebracht.
Um geeignete Einkristalle zur Röntgenstrukturanalyse zu gewinnen wurde 200 mg des Produktes in 0.5 mL Dichlormethan gelöst und mit 10 mL Cyclohexan überschichtet. Bereits nach einem Tag konnte Kristallwachstum im Bereich der ehemaligen Phasengrenze beobachtet werden. Nach sieben Tagen konnten geeignete Einkristalle entnommen werden. Ausbeute: 422 mg (0.9 mmol), 35%
¹H-NMR: (400 MHz, CDCl₃) δ [ppm] = 1.20 (d, J= 6.9 Hz, 12H), 2.42 (s, 6H), 3.21 (hept, J= 7 Hz, 2H), 6.37 (s, 2H), 7.18 (s, 2H)
¹³C-NMR: (100 MHz, CDCl₃) δ [ppm] = 20.76, 22.41, 27.96, 117.49, 126.39, 128.45, 134.16, 136.80, 152.70
Schmelzbereich: 175.3-175.8 °C

### Bis(3-chlor-6-hydroxy-5-methylphenyl)selen

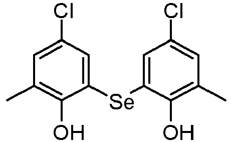

In einem 10 mL Rundkolben wurden 650 mg 4-Chlor-2-methylphenol (45 mmol) in 6.3 mL Pyridin gelöst, mit 506 mg Selendioxid (45 mmol) versetzt und im Ölbad auf 55 °C erhitzt. Nach zehn Tagen wurde die Reaktionslösung mit 50 mL Ethylacetat verdünnt und filtriert. Die organische Phase wurde zunächst zwei Mal mit je 40 mL 10%iger Salzsäure und zwei Mal mit je 40 mL Wasser gewaschen. Nach dem Trocknen über Magnesiumsulfat wurde das Lösungsmittel destillativ entfernt und das Rohprodukt mittels Säulenchromatographie gereinigt. Dabei wurde ein Säulenautomat der Firma *BÜCHI-Labortechnik GmbH,* Essen verwendet. Die Säulenlänge betrug 16 cm und der Durchmesser 6 cm. Als Eluent wurde Cyclohexan/Essigsäureethylester verwendet, wobei mit einem Essigsäureethylester Gradienten gearbeitet wurde: 1-5 % (über 15 min), 3-20% (über 20 min), 20-60% (20 min). Die Pumpgeschwindigkeit lag bei 50 mL/min. Das erhaltene Produkt wurde in Dichlormethan mit 5%iger Beimischung von Methanol gelöst und mit Cyclohexan überschichtet, um eine Kristallisation an der Grenzfläche zu ermöglichen. Es wurden farblose, nadelförmige Kristalle erhalten.
Ausbeute: 393 mg (1.0 mmol), 48 %
¹H-NMR: (400 MHz, CDCl₃) δ [ppm] = 2.25 (s, 6 H), 7.09-7.11 (m, 2H), 7.19-7.22 (m, 2H) ¹³C-NMR:(100 MHz, CDCl₃) δ [ppm] = 16.67, 125.57, 126.32, 126.91, 131.99, 132.24, 152.60

### Bis(2-hydroxy-3-methoxy-5-methylphenyl)selen

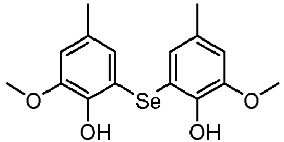

In einem 10 mL Rundkolben wurden 700 mg 4-Methylguaiacol (51 mmol) in 7,1 mL Pyridin gelöst, mit 0,856 g Selendioxid (77 mmol) versetzt und im Ölbad auf 55 °C erhitzt. Nach vier Tagen wurde die Reaktionslösung mit 50 mL Ethylacetat verdünnt und filtriert. Die organische Phase wurde zunächst zwei Mal mit je 40 mL 10%iger Salzsäure und zwei Mal mit je 40 mL Wasser gewaschen. Nach dem Trocknen über Magnesiumsulfat wurde das Lösungsmittel destillativ entfernt und das Rohprodukt mittels Säulenchromatographie gereinigt. Dabei wurde ein Säulenautomat der Firma *BÜCHI-Labortechnik GmbH,* Essen verwendet. Die Säulenlänge betrug 16 cm und der Durchmesser 6 cm. Als Eluent wurde Cyclohexan/Essigsäureethylester verwendet, wobei mit einem Essigsäureethylester Gradienten von 1-20% über 80 Minuten gearbeitet wurde. Die Pumpgeschwindigkeit lag bei 50 mL/min.
Um geeignete Einkristalle zur Röntgenstrukturanalyse zu gewinnen wurde 100 mg des Produktes in 0.3 mL Dichlormethan gelöst und mit 7 mL Cyclohexan überschichtet. Es wurden Plättchen aus klaren, leicht gelblichen Kristalle gewonnen.
Ausbeute: 167 mg (0.4 mmol), 19 %
¹H-NMR: (400 MHz, CDCl₃) δ [ppm] = 2.22 (s, 6 H), 3.85 (s, 6H), 6.26 (s, 2H), 6.64 (d, J= 1.6 Hz, 2H), 6.79 (dd, J1= 1.8Hz, J2= 0.7 Hz, 2H)
¹³C-NMR: (100 MHz, CDCl₃) δ [ppm] = 21.12, 56.22, 112.87, 114.96, 126.89, 130.30, 143.35, 156.52
Schmelzbereich: 146.5-146.8 °C

### Bis(3,5-dimethyl-4-hydroxyphenyl)selen

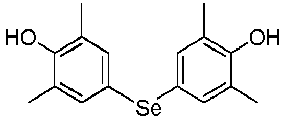

In einem Kulturröhrchen wurden 500 mg (4.1 mmol) 2,6-Dimethylphenol in 5.7 mL Pyridin gelöst und mit 250 mg (2.2 mmol) versetzt. Das Gemisch wurde in einem 55 °C heißen Stahlblock erhitzt. Nach 24 Stunden wurde das Reaktionsgemisch mit 40 mL Dichlormethan verdünnt und filtriert. Das Filtrat wurde zweimal mit je 30 mL Salzsäure (10%) und zweimal mit je 30 mL Wasser gewaschen. Die organische Phase wurde abgetrennt, mit Magnesiumsulfat getrocknet und vom Lösungsmittel befreit. Das so erhalten Rohprodukt wurde mittels Säulenchromatographie gereinigt. Dabei wurde ein Säulenautomat der Firma *BÜCHI-Labortechnik GmbH,* Essen verwendet. Die Säulenlänge betrug 16 cm und der Durchmesser 6 cm. Als Eluent wurde Cyclohexan/Essigsäureethylester verwendet, wobei mit einem Essigsäureethylester Gradienten gearbeitet wurde: 5-10 % (über 20min), 25-100% (über 5min). Das so erhaltene Produkt wurde in 20 mL Cyclohexan in der Siedehitze gelöst. Nach Abkühlung auf Raumtemperatur bildeten sich gelbliche Nadeln.

Ausbeute: 342 mg (1.1 mmol), 52%
¹H-NMR: (300 MHz, CDCl₃) δ [ppm] = 2.21 (s, 12H), 4.62 (s, 2H), 7.15 (s, 4H)
¹³C-NMR: (75 MHz, CDCl₃) δ [ppm] = 15.92, 121.48, 124.21, 133.71, 152.03 Schmelzbereich: 220.7-222.1 °C

### 3,3',5,5'-Tetra-tert-butylbiphenyl-2,2'-diol

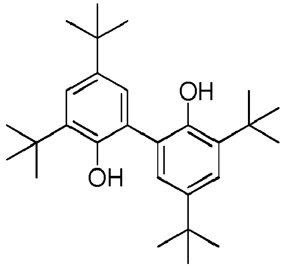

Die Durchführung der Reaktion erfolgt gemäß der allgemeinen Arbeitsvorschrift in einem verschraubbaren Reagenzglas. Dazu wurden 307 mg (1.5 mmol, 1.0 Äquiv.) *2,4-Di-tert-*butylphenol und 99 mg (0.8 mmol, 0.6 Äquiv.) Selendioxid in 0,5 mL Essigsäure gelöst und erhitzt.

¹H-NMR (400 MHz, CDCl₃):
δ (ppm) =7.39 (d, J=2.4 Hz, 2H), 7.11 (d, J=2.4, 2H), 5.21 (s, 2H), 1.45 (s, 18H), 1.32 (s, 18H).
¹³C-NMR (75 MHz, CDCl₃):
δ (ppm) =149.9, 143.0, 125.4, 124.9, 122.4, 35.4, 34.6, 31.7, 29.8.

Die Ergebnisse der oben beschrieben Reaktion, und Variationen derselben, sind in den folgenden Tabellen dargestellt. Die erfindungsgemäßen Verfahren sind hierbei mit * gekennzeichnet.

Folgende Verbindungsklassen werden in den Tabellen näher spezifiziert:

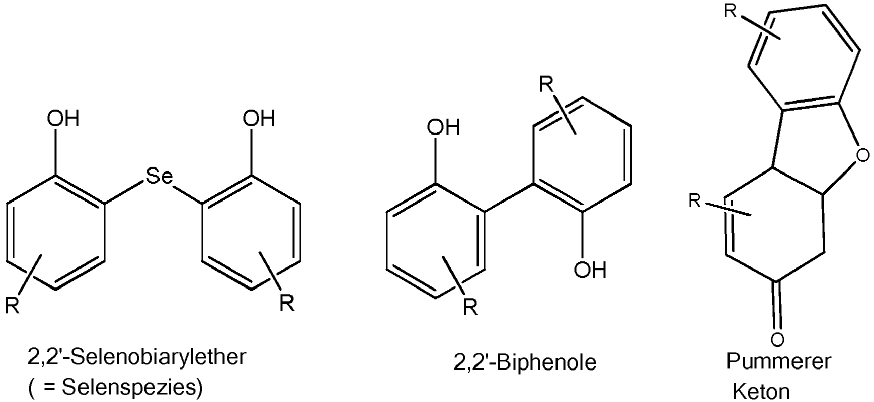

**Tabelle 1a: Oxidative Kupplung von 2,4-Dimethylphenol basische Bedingungen**

| | | | | | | |
|---|---|---|---|---|---|---|
| 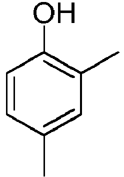 | | | | | | |

| Lösungsmittel | T [°C] | t [h] | pKb-Wert | Pummerer Keton [%] | Biphenol [%] | Selenspezies [%] |
|---|---|---|---|---|---|---|
| Pyridin* | 60 | 5 | 8,9 | - | - | 79,1 |
| Pyridin* | 85 | 5 | 8,9 | 2,6 | 13,1 | 59,6 |
| Pyridin* | 100 | 0,5 | 8,9 | 1,9 | 11,0 | 39,9 |
| Chinolin* | 60 | 7 | 9,2 | 0,6 | 1,9 | 28,6 |
| Triethylamin (trocken) | 80 | 4 | 3,3 | - | - | 1,8 |
| DMF | 85 | 5 | -1,1 | 4,2 | 19,1 | 18,8 |

Als weitere Stickstoffbase wurde 4-Dimethylaminopyridin (pKb = 4,8) verwendet. Die untersuchte Reaktionszeit betrug 1h, wobei gaschromatographisch weder das Biphenol, noch die Selenspezies nachgewiesen werden konnte.

Der Tabelle 1a kann man entnehmen, dass unter den erfindungsgemäßen Bedingungen der gewünschte 2,2'-Selenobiarylether immer als Hauptprodukt, im deutlichen Überschuss zu den Nebenprodukten, und in guter Ausbeute anfällt.

**Tabelle 1b: Oxidative Kupplung von 2,4-Dimethylphenol saure Bedingungen**

| 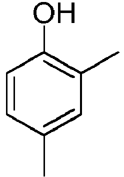 | | | | | | |
|---|---|---|---|---|---|---|
| Lösungsmittel | T [°C] | t [h] | pKs-Wert | Pummer er Keton [%] | Biphenol [%] | Selenspezies [%] |
| Essigsäure | 85 | 5 | 4,8 | 4,5 | 74,8 | 1,98 |
| Essigsäure | 60 | 1,5 | 4,8 | 1,8 | 39,8 | 8,0 |
| Trifluoressigsäure / Essigsäure (3:1) | 85 | 5 | 0,23 / 4,8 | 2,5 | 77,8 | 1,4 |
| Ameisensäure | 60 | 2 | 3,8 | 1,8 | 85,4 | - |
| Methansulfonsäure | 85 | 5 | -2,6 | - | 3,9 | 6,1 |
| p-Toluolsulfonsäure | 85 | 5 | -2,8 | - | 15,0 | - |

Der Tabelle 1 b kann man entnehmen, dass unter sauren Bedingungen jeweils das Biphenol als Hauptprodukt anfällt. Die einzige Ausnahme bildet Methansulfonsäure, wobei hier die Ausbeute an der Selenspezies sehr gering ist.

**Tabelle 2a: Oxidative Kupplung von 2,4-Di-tert-butylphenol basische Bedingungen**

| | | | | | |
|---|---|---|---|---|---|
| 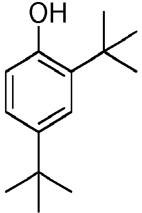 | | | | | |

| Lösungsmittel | T [°C] | t [h] | pKb-Wert | Biphenol [%] | Selenspezies [%] |
|---|---|---|---|---|---|
| Pyridin* | 40 | 24 | 8,9 | 20,6 | 46,8 |
| Pyridin* | 60 | 7 | 8,9 | 10,1 | 30,4 |

Unter den erfindungsgemäßen basischen Bedingungen entsteht wieder die Selenspezies als Hauptprodukt der Reaktion.

**Tabelle 2b: Oxidative Kupplung von 2,4-Di-tert-butylphenol saure Bedingungen**

| | | | | | |
|---|---|---|---|---|---|
| 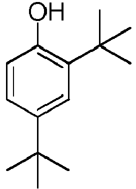 | | | | | |

| Lösungsmittel | T [°C] | t [h] | pKs-Wert | Biphenol [%] | Selenspezies [%] |
|---|---|---|---|---|---|
| Essigsäure | 50 | 18 | 4,8 | 29,5 | 25,2 |
| Essigsäure | 85 | 1 | 4,8 | 25,9 | 23,1 |
| Essigsäure | 105 | 0,2 | 4,8 | 75,1 | 2,6 |
| Ameisensäure | 70 | 1 | 3,8 | 46,9 | 7,6 |

Unter sauren Bedingungen hingegen ist das unerwünschte Biphenol das Hauptprodukt der Reaktion.

**Tabelle 3a: Oxidative Kupplung von 2-tert-Butyl-4-methylphenol basische Bedingungen**

| | | | | | |
|---|---|---|---|---|---|
| 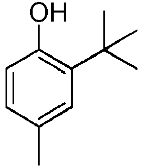 | | | | | |

| Lösungsmittel | T [°C] | t [h] | pKb-Wert | Biphenol [%] | Selenspezies [%] |
|---|---|---|---|---|---|
| Pyridin* | 40 | 24 | 8,9 | 7,2 | 61,5 |
| Pyridin* | 60 | 7 | 8,9 | 1,6 | 32,2 |
| Pyridin* | 85 | 1,5 | 8,9 | 6,1 | 32,5 |
| Pyridin* | 100 | 0,5 | 8,9 | 4,5 | 28,9 |

Auch aus der Tabelle 3a ist wiederum ersichtlich, dass die erfindungsgemäßen basische Bedingungen zur gewünschten Selenspezies führt. Diese wird im deutlichen Überschuss gegenüber dem unerwünschten Biphenol erhalten.

**Tabelle 3b: Oxidative Kupplung von 2-tert-Butyl-4-methylphenol saure Bedingungen**

| | | | | | |
|---|---|---|---|---|---|
| 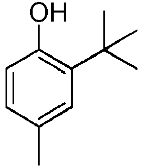 | | | | | |

| Lösungsmittel | T [°C] | t [h] | pKs-Wert | Biphenol [%] | Selenspezies [%] |
|---|---|---|---|---|---|
| Essigsäure | 50 | 18 | 4,8 | 34,2 | 19,8 |
| Essigsäure | 85 | 1,5 | 4,8 | 63,7 | 4,0 |
| Essigsäure | 100 | 0,4 | 4,8 | 53,2 | 2,1 |

Unter sauren Bedingungen hingegen stellt die angestrebte Selenspezies wiederum nur das Nebenprodukt dar.

Die in den Tabellen 1a bis 3b zusammengefassten Ergebnisse zeigen eindeutig, dass das erfindungsgemäße Verfahren die zuvor definierte Aufgabenstellung erfüllt. Das erfindungsgemäße Verfahren stellt eine Syntheseroute dar, mit welcher 2,2'-Selenobiarylether selektiv, in guter Ausbeute hergestellt werden können. Des Weiteren lässt sich das erfindungsgemäße Verfahren auch großtechnisch verwirklichen. Die Phenole werden in einem einzigen Reaktionsschritt direkt zu den entsprechenden 2,2'-Selenobiarylether umgesetzt.

## Patentansprüche

1. Verfahren zur Herstellung von 2,2'-Selenobiarylethern oder 4,4'-Selenobiarylethern umfassend die Verfahrensschritte:
a) Zugabe eines ersten Phenols zum Reaktionsgemisch,
b) Zugabe eines zweiten Phenols zum Reaktionsgemisch,
c) Zugabe von Selendioxid zum Reaktionsgemisch,
d) Zugabe einer Base mit einem pKb-Wert im Bereich von 8 bis 11 zum Reaktionsgemisch,
e) Einstellen der Reaktionstemperatur des Reaktionsgemisches, so dass das erste Phenol und das zweite Phenol zu einem 2,2'-Selenobiarylether oder 4,4'-Selenobiarylether umgesetzt werden.

2. Verfahren nach Anspruch 1,
wobei es sich bei dem ersten Phenol in Verfahrensschritt a) um eine Verbindung gemäß der allgemeinen Formel **I** handelt: 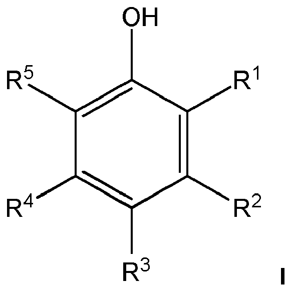 wobei R¹, R², R³, R⁴, R⁵ jeweils unabhängig voneinander ausgewählt sind aus:
- H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl,
zwei benachbarte Reste können des Weiteren zu einem kondensierten System miteinander verbunden sein,
wobei die genannten Alkyl- und Arylgruppen substituiert sein können,
und mindestens R¹ oder R⁵ gleich -H ist.

3. Verfahren nach Anspruch 2,
wobei R¹, R², R³, R⁴, R⁵ jeweils unabhängig voneinander ausgewählt sind aus:
- H, -(C₁-C₁₂)-Alkyl, -O-(C₁-C₁₂)-Alkyl, -(C₆-C₂₀)-Aryl, -O-(C₆-C₂₀)-Aryl,
wobei die genannten Alkyl- und Arylgruppen substituiert sein können,
und mindestens R¹ oder R⁵ gleich -H ist.

4. Verfahren nach einem der Ansprüche 2 oder 3,
wobei R¹, R³, R⁵ jeweils unabhängig voneinander ausgewählt sind aus:
- H, -(C₁-C₁₂)-Alkyl,
wobei die genannten Alkylgruppen substituiert sein können,
und mindestens R¹ oder R⁵ gleich -H ist.

5. Verfahren nach einem der Ansprüche 1 bis 4,
wobei es sich bei dem zweiten Phenol in Verfahrensschritt b) um eine Verbindung gemäß der allgemeinen Formel **II** handelt: 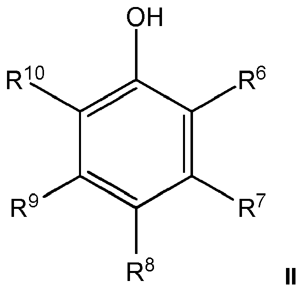 wobei R⁶, R⁷, R⁸, R⁹, R¹⁰ jeweils unabhängig voneinander ausgewählt sind aus:
- H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl,
zwei benachbarte Reste können des Weiteren zu einem kondensierten System miteinander verbunden sein,
wobei die genannten Alkyl- und Arylgruppen substituiert sein können,
und mindestens R⁶ oder R¹⁰ gleich -H ist.

6. Verfahren nach Anspruch 5,
wobei R⁶, R⁷, R⁸, R⁹, R¹⁰ jeweils unabhängig voneinander ausgewählt sind aus:
- H, -(C₁-C₁₂)-Alkyl, -O-(C₁-C₁₂)-Alkyl, -(C₆-C₂₀)-Aryl, -O-(C₆-C₂₀)-Aryl,
wobei die genannten Alkyl- und Arylgruppen substituiert sein können,
und mindestens R⁶ oder R¹⁰ gleich -H ist.

7. Verfahren nach einem der Ansprüche 5 oder 6,
wobei R⁶, R⁸, R¹⁰ jeweils unabhängig voneinander ausgewählt sind aus:
- H, -(C₁-C₁₂)-Alkyl,
wobei die genannten Alkylgruppen substituiert sein können,
und mindestens R⁶ oder R¹⁰ gleich -H ist.

8. Verfahren nach einem der Ansprüche 1 bis 7,
wobei das erste Phenol dem zweiten Phenol entspricht.

9. Verfahren nach einem der Ansprüche 1 bis 8,
wobei das Selendioxid in Verfahrensschritt c) in einem molaren Verhältnis bezogen auf die Summe des ersten und des zweiten Phenols zugegeben wird, welches in einem Bereich von 0,25 bis 1,5 liegt.

10. Verbindung gemäß der Formel:

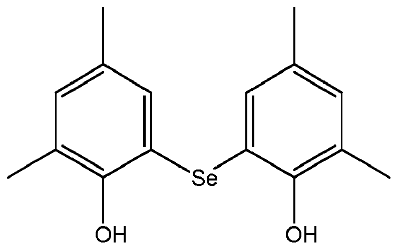

11. Verfahren nach Anspruch 1,
wobei es sich bei dem ersten Phenol in Verfahrensschritt a) um eine Verbindung gemäß der allgemeinen Formel **III** handelt: 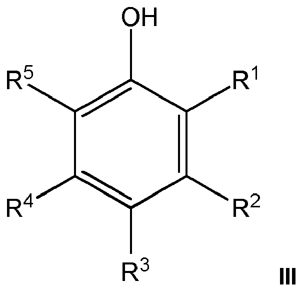 wobei R¹, R², R³, R⁴, R⁵ jeweils unabhängig voneinander ausgewählt sind aus:
- H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl,
zwei benachbarte Reste können des Weiteren zu einem kondensierten System miteinander verbunden sein,
wobei die genannten Alkyl- und Arylgruppen substituiert sein können,
und R³ gleich -H ist.

12. Verfahren nach Anspruch 11,
wobei R¹, R², R³, R⁴, R⁵ jeweils unabhängig voneinander ausgewählt sind aus:
- H, -(C₁-C₁₂)-Alkyl, -O-(C₁-C₁₂)-Alkyl, -(C₆-C₂₀)-Aryl, -O-(C₆-C₂₀)-Aryl,
wobei die genannten Alkyl- und Arylgruppen substituiert sein können,
und R³ gleich -H ist.

13. Verfahren nach einem der Ansprüche 11 oder 12,
wobei R¹, R³, R⁵ jeweils unabhängig voneinander ausgewählt sind aus:
- H, -(C₁-C₁₂)-Alkyl,
wobei die genannten Alkylgruppen substituiert sein können,
und R³ gleich -H ist.

14. Verfahren nach einem der Ansprüche 11 bis 13,
wobei es sich bei dem zweiten Phenol in Verfahrensschritt b) um eine Verbindung gemäß der allgemeinen Formel **IV** handelt: 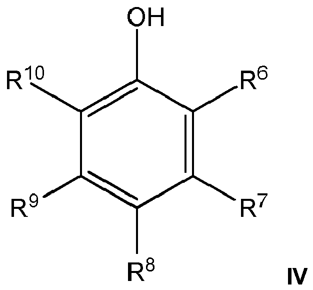 wobei R⁶, R⁷, R⁸, R⁹, R¹⁰ jeweils unabhängig voneinander ausgewählt sind aus:
- H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl,
zwei benachbarte Reste können des Weiteren zu einem kondensierten System miteinander verbunden sein,
wobei die genannten Alkyl- und Arylgruppen substituiert sein können,
und R⁸ gleich -H ist.

15. Verfahren nach Anspruch 14,
wobei R⁶, R⁷, R⁸, R⁹, R¹⁰ jeweils unabhängig voneinander ausgewählt sind aus:
- H, -(C₁-C₁₂)-Alkyl, -O-(C₁-C₁₂)-Alkyl, -(C₆-C₂₀)-Aryl, -O-(C₆-C₂₀)-Aryl,
wobei die genannten Alkyl- und Arylgruppen substituiert sein können,
und R⁸ gleich -H ist.

16. Verfahren nach einem der Ansprüche 14 oder 15,
wobei R⁶, R⁸, R¹⁰ jeweils unabhängig voneinander ausgewählt sind aus:
- H, -(C₁-C₁₂)-Alkyl,
wobei die genannten Alkylgruppen substituiert sein können,
und R⁸ gleich -H ist.

17. Verfahren nach einem der Ansprüche 14 bis 16,
wobei das erste Phenol dem zweiten Phenol entspricht.

18. Verfahren nach einem der Ansprüche 14 bis 17,
wobei das Selendioxid in Verfahrensschritt c) in einem molaren Verhältnis bezogen auf die Summe des ersten und des zweiten Phenols zugegeben wird, welches in einem Bereich von 0,25 bis 1,5 liegt.

19. Verbindung gemäß der Formel:
